# EUROPÄISCHE PATENTSCHRIFT

(11) **EP 1 357 350 B1**
(45) Veröffentlichungstag und Bekanntmachung des Hinweises auf die Patenterteilung: **29.06.2005**
(21) Anmeldenummer: 03090116.9
(22) Anmeldetag: 23.04.2003
(51) Int. Cl.: G01B 5/02, G01N 3/40, G01N 33/15

(54) **Oblongzentrierung und ( elektrische ) Breitenmessung ( Tablette ) mit Prüfbacken mit Doppelexzenter**
Oblong alignment and ( electrical ) measurement of width ( medicinal tablet ) with testing chucks and double eccentric
Alignement oblong et mesure (électrique ) de largeur ( comprimé ) avec des mâchoires de test et excentrique double

(30) Priorität: 24.04.2002 DE 20206782 U
(43) Veröffentlichungstag der Anmeldung: 29.10.2003
(73) Patentinhaber: Charles Ischi AG, 4528 Zuchwil (CH)
(72) Erfinder: Ischi, Charles, 4500 Solothum (CH)
(74) Vertreter: Meissner, Peter E., Dipl.-Ing.

(56) Entgegenhaltungen:
- EP-A- 0 987 527
- DE-A- 19 744 227
- DE-C- 10 024 970
- US-A- 4 884 463

## Beschreibung

Die Erfindung betrifft eine Vorrichtung, mit der es ermöglicht werden soll, bei Tablettenprüfsystemen die Prüflinge vor der Prüfung in bestimmter Weise zu positionieren und während des Prüfvorgangs zu führen, um ein Wegdrehen von länglichen Prüfobjekten bei der Bruchfestigkeitsprüfung zu verhindern, sowie eine Breiten- bzw. Dickenmessung der Prüflinge auszuführen (DE-CI-100 24 970).
Bisher war es bekannt, die Prüflinge entweder manuell, durch Transportsterne oder Transportrechen für die Prüfung zu positionieren. Diese Art der Positionierung ist jedoch zu ungenau und schließt auch keine Führung während des Prüfvorgangs sowie keine Breitenmessung der Prüflinge ein.

Der Erfindung lag deshalb die Aufgabe zugrunde, eine Vorrichtung zur ortsgenauen Positionierung und Führung der Prüflinge in Tablettenprüfsystemen zu schaffen.
Außerdem sollte ohne wesentlichen zusätzlichen Aufwand eine Breitenmessung der Prüflinge möglich sein.
Die Lösung der Aufgabe erfolgt entsprechend den Merkmalen des Anspruchs 1.
Die Unteransprüche geben zweckmäßige Ausgestaltungen der Erfindungsmerkmale wieder.

Die Erfindung soll an einem Ausführungsbeispiel anhand der Figuren erläutert werden.
Es zeigen:
- Fig. 1:: eine Prinzipdarstellung der erfindungsgemäßen Vorrichtung,
- Fig. 2:: den Doppelexzenter der Zentriervorrichtung in der Draufsicht.

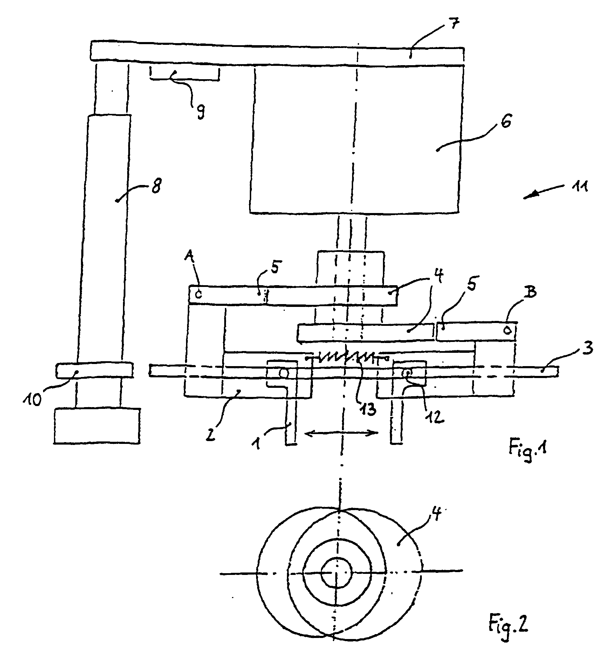

Soll ein Prüfobjekt zentriert oder vermessen werden, schaltet eine Interfaceplatine das Steuerprogramm des Prüfsystems nach dem Sterntakt der Prüfanordnung (in Fig. 1 nicht dargestellt) auf den Antrieb der Zentriervorrichtung um. Ein Ausheber 9 hebt den mit den Enden der Messwellen 12 auf der Platte 10 geparkten Sensorkopf 11 von der Platte 10 ab (in Fig. 1 ist der Sensorkopf um 90° gedreht dargestellt) und der Nullpunkt der Vorrichtung wird ermittelt. Letzteres ist notwendig, da der Antrieb der Vorrichtung während des Sterntaktes des Prüfsystems stromlos ist und seine Position verliert.
Durch Drehung des Schrittmotors 6 und des an dessen Motorwelle angebrachten Doppelexzenters 4 werden die auf der gemeinsamen Welle 3 gleitenden Schlitten 2 über die Kontaktplatten 5 gegen die Wirkung der Zugfeder 13 so weit gegenläufig verfahren, dass die an den Schlitten 2 angebrachten Messwellen 12 auseinanderfahren und der an der Traverse 7 angebrachte Sensorkopf 11 mittels der Linearführung 8 so weit abgesenkt werden kann, bis die an den Messwellen 12 angebrachten Prüfbacken 1 das Niveau der Prüflinge erreichen. Sodann dreht der Schrittmotor 6 in entgegengesetzter Richtung so weit, bis die Prüfbacken 1 unter der Wirkung der Zugfeder 13 kraftschlüssig am Prüfobjekt anliegen.
Bei Weiterfahren des Schrittmotors 6 öffnet sich der Kontakt zwischen den Exzentern des Doppelexzenters 4 und den Kontaktplatten 5. Dadurch wird ein durch Anlegen einer elektrischen Spannung an die Anschlußpunkte A, B der Kontaktplatten 5 bis dahin über die Kontaktstellen zwischen Exzenter und Kontaktplatte 5 geflossener Strom unterbrochen. Bei Stromunterbrechung erfolgt die Breitenmessung, d.h. es wird die Winkelstellung des Schrittmotors 6 erfasst, die entsprechend einer vorher durchgeführten Justage ein Ausdruck für die Breite des Prüflings ist.

Bei aktiver Zentrierfunktion, d.h. bei der eigentlichen Bruchfestigkeitsprüfung, öffnen sich die Prüfbacken 1 einen Spalt weit, um den Messvorgang physikalisch nicht zu beeinflussen, und der Härtetester des Prüfsystems führt seine Prüfung durch. Durch die Prüfbacken 1 wird der Prüfling dabei so weit geführt, dass er sich nicht wegdrehen kann.
Nach Abschluß der Prüfung bzw. Messung öffnen sich die Prüfbacken 1 und der Sensorkopf 11 fährt in die Parkposition zurück, um die Bahn für den nächsten Sterntakt des Prüfsystems freizugeben.
Die Prüfbacken 1 sind zur parallelen Ausrichtung zueinander auf den Messwellen 12 drehbar angeordnet. Der Schrittmotor 6 und die Antriebsmechanik der Prüfbacken 1 sind in einem Gehäuse untergebracht, wobei ein Verdrehschutz der Schlitten 2 gleichzeitig eine Abdichtung der Antriebseinheit nach außen bildet.
Die Justierung der Anzeige für die Breitenmessung des Prüflings, d.h. die Festlegung der Beziehung zwischen der Winkelstellung des Schrittmotors (6) und dem Abstand der Prüfbacken (1) voneiander, erfolgt mittels zweier Endmaße, deren Größe im oberen und unteren Bereich des nutzbaren Meßbereichs liegt.

## Patentansprüche

1. Vorrichtung zur Oblongzentrierung und Breitenmessung für Tablettenprüfsysteme, die im Bereich der Transportbahn der Prüflinge über der Härtemessstation montierbar ist, bei der zwei Prüfbacken (1) durch eine Antriebsmechanik zusammengeführt werden, wodurch die Prüfbacken (1) den Prüfling zwischen sich einschließen und ausrichten, sodann nach geringfügigem Öffnen der Prüfbacken eine Führung gegen Wegdrehen des Prüflings bei der Bruchfestigkeitsprüfung bilden und wobei Mittel vorgesehen sind, die den Abstand der Prüfbacken (1) bei deren Anliegen am Prüfling als Maß für dessen Breite erfassen.

2. Vorrichtung nach Anspruch 1,
**dadurch gekennzeichnet,**
**dass** die Prüfbacken (1) über Messwellen (12) an Schlitten (2) befestigt sind, die mittels Gleitlager auf einer gemeinsamen Welle (3) geführt und mittels einer Zugfeder (13) zueinander gezogen werden, dass der Antrieb der Schlitten (2) aus einem Schrittmotor (6) besteht, der über seine Welle einen Doppelexzenter (4) dreht, welcher über Kontaktplatten (5) die Schlitten (2) gegenläufig verfährt.

3. Vorrichtung nach Anspruch 2,
**dadurch gekennzeichnet,**
**dass** an den beiden Kontaktplatten (5) elektrische Anschlüsse (A, B) vorgesehen sind, über die bei Anliegen einer elektrischen Spannung ein Strom durch die Kontaktplatten (5) und die Exzenter des Doppelexzenters (4) fließt, der bei Anliegen der Prüfbacken (1) an dem Prüfling und Öffnen des Kontaktes zwischen den Kontaktplatten (5) und dem jeweiligen Exzenter bei Weiterfahren des Schrittmotors (6) unterbrochen wird und somit aus der Winkelstellung des Schrittmotors (6) in diesem Augenblick der Stromunterbrechung ein Maß für die Breite des Prüflings abgeleitet und über ansich bekannte Mittel angezeigt wird.

4. Vorrichtung nach Anspruch 2 oder 3,
**dadurch gekennzeichnet,**
**dass** das gesamte System des Schrittmotors (6) mit den Prüfbacken (1) und deren Antrieb als Sensorkopf (11) über eine Traverse (7) an einer senkrechten Linearführung (8) angeordnet ist, die ein Schwenken und mittels eines Aushebers (9) ein Heben und Senken des Sensorkopfs (11) ermöglicht.

5. Vorrichtung nach einem der Ansprüche 2 bis 4,
**dadurch gekennzeichnet,**
**dass** die Prüfbacken (1) zur parallelen Ausrichtung zueinander auf den Messwellen (12) drehbar sind.

6. Vorrichtung nach Anspruch 4,
**dadurch gekennzeichnet,**
**dass** an der Linearführung (8) eine waagerechte Platte (10) befestigt ist, auf der die Enden der Messwellen (12) in einer Parkstellung des Sensorkopfes (11) auflegbar sind.

7. Vorrichtung nach Anspruch 3.
**dadurch gekennzeichnet,**
**dass** die Justierung der Anzeige für die Breitenmessung, d.h. die Winkelstellung des Schrittmotors (6) in Abhängigkeit vom Abstand der Prüfbacken (1) voneiander, mittels zweier Endmaße, deren Größe im oberen und unteren Bereich des nutzbaren Meßbereichs liegt, erfolgt.

8. Vorrichtung nach einem der vorangehenden Ansprüche,
**dadurch gekennzeichnet,**
**dass** Mittel vorgesehen sind, die es gestatten, die Öffnungsweite der Prüfbacken (1) beliebig einzustellen, damit diese bei der Bruchfestigkeitsprüfung eine Führungsfunktion für die Prüflinge übemehmen können.

9. Vorrichtung nach einem der vorangehenden Ansprüche,
**dadurch gekennzeichnet,**
**dass** der Sensorkopf (11) in einem Gehäuse untergebracht ist, wobei ein Verdrehschutz der Schlitten (2) gleichzeitig eine Abdichtung der Antriebseinheit nach außen bildet.

10. Vorrichtung nach einem der vorangehenden Ansprüche,
**dadurch gekennzeichnet,**
**dass** das Programm für die Vorrichtung als optional aktivierbares Software-Modul im Haupteprom der Prüfsystem-Steuerung integriert ist.

## Claims

1. A device for oblong centring and width measurement for tablet testing systems, which is mountable in the region of the conveyor path of the test pieces over the hardness measuring bay, wherein two test jaws (1) are brought together by a drive mechanism, whereby the test jaws (1) enclose and align the test piece between them, and then after slight opening of the test jaws form a guide to prevent the test piece from turning away in the breaking strength test, and wherein means are provided for determining the distance between the test jaws (1) when these are applied to the test piece as a measure of its width.

2. A device according to claim 1, **characterised in that** the test jaws (1) are fixed to slides (2) via measuring shafts (12), which are guided on a common shaft (3) by means of slide bearings and are drawn together by means of a tension spring (13), and **in that** the drive of the slides (2) consists of a stepper motor (6), which via its shaft rotates a double cam (4), which drives the slides (2) in opposite directions via contact plates (5).

3. A device according to claim 2, **characterised in that** electrical terminals (A, B) are provided on both contact plates (5), via which terminals, upon the application of an electric voltage, a current flows through the contact plates (5) and the cams of the double cam (4), which is interrupted when the test jaws (1) are applied to the test piece and the contact between the contact plates (5) and the respective cam is broken, with the stepper motor (6) continuing to operate, and thus from the angular position of the stepper motor (6) at this moment of breaking of the current a measure of the width of the test piece is derived and is displayed via means known *per se.*

4. A device according to claim 2 or 3, **characterised in that** the entire system of the stepper motor (6) together with the test jaws (1) and their drive is disposed on a vertical linear guide (8) via a crosspiece (7) as a sensor head (11), the linear guide permitting pivoting and, by means of a lifter (9), lifting and lowering of the sensor head (11).

5. A device according to one of claims 2 to 4, **characterised in that** the test jaws (1) are rotatable for parallel alignment relative to one another on the measuring shafts (12).

6. A device according to claim 4, **characterised in that** on the linear guide (8) a horizontal plate (10) is fixed, on which the ends of the measuring shafts (12) may be laid in a parking position of the sensor head (11).

7. A device according to claim 3, **characterised in that** adjustment of the display for width measurement, i.e. the angular position of the stepper motor (6) as a function of the distance of the test jaws (1) from one another, is effected by means of two end measures, whose amount lies in the upper and lower region of the useful measuring range.

8. A device according to one of the preceding claims, **characterised in that** means are provided which permit the test jaws (1) to be set at any width of aperture in order that these can act as a guide for the test pieces during the breaking strength test.

9. A device according to one of the preceding claims, **characterised in that** the sensor head (11) is accommodated in a housing, wherein a shield to protect against twisting of the slides (2) simultaneously forms an external seal of the drive unit.

10. A device according to one of the preceding claims, **characterised in that** the programme for the device is incorporated in the main EPROM of the test system control as an optionally activatable software unit.

## Revendications

1. Dispositif de centrage oblong et de mesure de la largeur pour systèmes de contrôle de billettes, qui peut être monté dans la zone de la voie de transport des éprouvettes au-dessus de la station de mesure de dureté, dans lequel deux mâchoires de contrôle (1) sont amenées l'une vers l'autre par un mécanisme d'entraînement, moyennant quoi les mâchoires de contrôle (1) enferment entre elles et orientent l'éprouvette, forment ensuite après légère ouverture des mâchoires de contrôle un guidage pour éviter la déviation de l'éprouvette lors de l'essai de résistance à la rupture, et des moyens étant prévus pour déterminer l'écart entre les mâchoires de contrôle (1) lorsqu'elles reposent contre l'éprouvette en tant que mesure de la largeur de celle-ci.

2. Dispositif selon la revendication 1,
**caractérisé en ce que** les mâchoires de contrôle (1) sont fixées par l'intermédiaire d'arbres de mesure (12) sur des coulisseaux (2) qui sont guidés au moyen de paliers tisses sur un arbre commun (3) et sont tirés l'un vers l'autre au moyen d'un ressort de traction (13), **en ce que** t'entraînement des coulisseaux (2) est constitué d'un moteur pas à pas (6) qui fait tourner par l'intermédiaire de son arbre un excentrique double (4) qui déplace les coulisseaux (2) en sens contraire par l'intermédiaire de plaques de contact (5).

3. Dispositif selon la revendication 2,
**caractérisé en ce que** sur les deux plaques de contact (5) des raccordements électriques (A, B) sont prévus, par l'intermédiaire desquels un courant passe par les plaques de contact (5) et les excentriques de l'excentrique double (4) en présence d'une tension électrique, ledit courant étant interrompu lors du contact des mâchoires de contrôle (1) contre l'éprouvette et..-de l'ouverture du contact entre les plaques de contact (5) et l'excentrique correspondant lorsque le moteur pas à pas (6) continue à marcher et une mesure de la largeur de l'éprouvette est ainsi déduite à partir de la position angulaire du moteur pas à pas (6) à ce moment de l'interruption de courant et est indiquée par des moyens connus en soi.

4. Dispositif selon la revendication 2 ou 3,
**caractérisé en ce que** tout le système du moteur pas à pas (6) avec les mâchoires de contrôle (1) et leur entraînement est agencé comme tête de capteur (11) par l'intermédiaire d'une traverse (7) sur un guidage linéaire vertical (8) qui permet de faire pivoter et, au moyen d'un élévateur (9), de lever et d'abaisser la tête de capteur (11).

5. Dispositif selon l'une des revendications 2 à 4,
**caractérisé en ce que** les mâchoires de contrôle (1) peuvent tourner sur les arbres de mesure (12) pour l'orientation parallèle l'une par rapport à l'autre.

6. Dispositif selon la revendication 4,
**caractérisé en ce qu'**une plaque horizontale (10) est fixée sur le guidage linéaire (8), sur laquelle les extrémités des arbres de mesure (12) peuvent être posées dans une position d'arrêt de la tête de capteur (11).

7. Dispositif selon la revendication 3,
**caractérisé en ce que** le réglage de l'indication de mesure de la largeur, c'est-à-dire la position angulaire du moteur pas à pas (6), a lieu en fonction de l'écart des mâchoires de contrôle (1) entre elles, au moyen de deux dimensions d'extrémité dont la grandeur se situe dans la zone supérieure et inférieure de la plage de mesure utile.

8. Dispositif selon l'une des revendications précédentes,
**caractérisé en ce que** des moyens sont prévus pour permettre de régler à volonté la largeur d'ouverture des mâchoires de contrôle (1) afin que celles-ci puissent assurer une fonction de guidage des éprouvettes lors de l'essai de résistance à la rupture.

9. Dispositif selon l'une des revendications précédentes,
**caractérisé en ce que** la tête de capteur (11) est logée dans un boîtier, une protection antitorsion des coulisseaux (2) formant en même temps vers l'extérieur une étanchéité de l'unité d'entraînement.

10. Dispositif selon l'une des revendications précédentes,
**caractérisé en ce que** le programme du dispositif est intégré dans la mémoire morte programmable principale de la commande du système de contrôle en tant que module de logiciel pouvant être activé au choix.
